# EUROPEAN PATENT SPECIFICATION

(11) **EP 1 220 659 B1**
(45) Date of publication and mention of the grant of the patent: **01.09.2004**
(21) Application number: 00968068.7
(22) Date of filing: 11.10.2000
(51) Int. Cl.: A61K 9/22, A61K 9/02, A61K 9/52

(54) **PHARMACEUTICAL PRODUCTS AND METHODS OF FABRICATION THEREFOR**
PHARMAZEUTISCHE PRODUKTE UND VERFAHREN ZU DEREN HERSTELLUNG
PRODUITS PHARMACEUTIQUES ET LEURS METHODES DE FABRICATION

(30) Priority: 15.10.1999 GB 9924334
(43) Date of publication of application: 10.07.2002
(73) Proprietor: pSiMedica Limited, Worcestershire, WR14 3SZ (GB)
(72) Inventor: CANHAM, Leigh Trevor, Malvern Worcs WR14 3PS (GB); SAPSFORD, Guy, Malvern Worcs WR14 3PS (GB)
(74) Representative: Bowdery, Anthony Oliver
(86) International application number: PCT/GB2000/003894
(87) International publication number: WO 2001/028529

(56) References cited:
- WO-A-97/06101
- WO-A-98/00107
- WO-A-99/53898
- DESAI TEJAL A ET AL: "Microfabricated immunoisolating biocapsules." BIOTECHNOLOGY AND BIOENGINEERING, vol. 57, no. 1, 5 January 1998 (1998-01-05), pages 118-120, XP002163449 ISSN: 0006-3592

## Description

This invention relates to orally administrable pharmaceutical products that comprise porous and/or polycrystalline silicon and to suppositories that comprise porous and/or polycrystalline silicon. The invention further relates to methods of fabricating such orally administrable products and suppositories.

Drugs are frequently taken orally by a patient, the drug being absorbed by the digestive system of the patient. Orally administered pharmaceutical products include tablets, pellets, capsules, powders, suspensions, and solutions. Capsules comprise a shell that contains the drug; the size and shape of the capsule being such that the patient can swallow the capsule. Capsules can be used to deliver beneficial substances other than drugs, such as nutrients and minerals. Usually the shell comprises gelatine and is formed from two halves, the halves being bonded together. The gelatine is rapidly disfigured in the gastric tract permitting the gastric fluids to penetrate the shell and reach the drug.

Tablets and pellets comprise a sample of solid material having a shape and size that allows a patient to swallow the tablet or pellet. Prior art tablets and pellets are typically prepared by compression or moulding of the solid material; the material may comprise a beneficial substance combined with one or more of: a diluent, a coating, a colorant, and a disintegrant. Diluents are fillers used in preparing tablets or pellets of the appropriate size and consistency; disintegrants are used for the break up and separation of the tablet's or pellet's compressed ingredients.

There are several different types of tablet and pellet coating. Excipient coatings are used to make the beneficial substance, or other material from which the tablet or pellet is comprised, more palatable to the patient. For example the beneficial substance may have an unpleasant or bitter taste which could be concealed by the coating.

A further type of coating protects the beneficial substance as it passes through the gastric tract and breaks up in the intestine to release the beneficial substance. These are termed enteric coatings and are of value if the intestine is the site at which the drug is to be delivered. Examples of such drugs are those used in the treatment of Crohn's disease, ulcerative colitis, constipation, colorectal cancer, spastic colon, and irritable bowel syndrome, which are all best delivered in the colon. For the purposes of this specification, this type of drug will be referred to as a colonic drug. Enteric coatings are often required because many beneficial substances are attacked in the gastric environment.

The pH of the gastrointestinal tract is low in the stomach and increases along its length. One type of prior art enteric coating therefore comprises methacrylic resins that are insoluble at low pH and soluble at high pH. The resin dissolves in the intestine so that the beneficial substance is released. Unfortunately intestinal pH is not predictably stable making this form of delivery unreliable. The colon is inhabited by many bacteria including bacteria that are known to reduce the azo bond. A further type of enteric coating therefore comprises polymers having azo bonds; the polymer decomposing in the intestine. However, microbial digestion and gastrointestinal transit times can vary greatly. For a human patient drugs taken before meals typically pass through the stomach within one hour, but when taken after a meal the drug can take up to ten hours. Transit times are also affected by the age and health of the patient. Variations in transit time and microbial digestion can greatly affect the location and rate of delivery associated with azo polymer coatings.

The fabrication of existing forms of orally administered pharmaceutical product is a relatively inexpensive and straightforward process. For example the process may involve insertion of the beneficial substance into a gelatine shell to form a capsule, or it may involve forming a drug and filler into a tablet. However, materials currently used in known orally administered pharmaceuticals are relatively difficult to engineer in relation to control of rate and location of drug release. Variations, in gastrointestinal conditions, from human patient to human patient are not easily accommodated by existing orally administered products.

In addition to oral administration, it is also possible to deliver beneficial substances by means of a suppository via other body orifices. A suppository is a solid dosage form intended for insertion into body orifices where they melt, soften, or dissolve to release the beneficial substance. Suppositories are commonly employed rectally, vaginally, and occasionally urethrally. The shape and size of the suppository must be such that it is capable of being easily inserted into the intended body orifice without causing undue distension, and once inserted, it must be retained for the appropriate period of time. However, similar problems also apply to these forms of administration: existing products do not provide sufficient control over the rate of release of the beneficial substance contained in the suppository.

Two materials that have proved relatively easy to engineer, with respect to biological applications, are porous and polycrystalline silicon. GB 9808052.6 describes implants, formed from porous and polycrystalline silicon, which release a beneficial substance into the body of the patient. The application describes the formation of the porous silicon by anodising bulk crystalline silicon. The application also describes the fabrication of structures, using micromachining techniques, that allow the controlled release of the beneficial substance. The formation of polycrystalline silicon is discussed in GB 9914298.6 and involves the growth of a silicon oxide layer on silicon and followed by deposition of polycrystalline silicon; the deposition being achieved by pyrolysis of SiH₄. Such anodising, deposition, and micromachining techniques are expensive relative to standard techniques for forming tablets, pellets, and capsules. The implants of GB 9808052.6 operate by the corrosion of porous silicon. Such corrosion is disclosed, in simulated body fluid, in GB 9914298.6.

WO 99/53898 A claims the priority of GB 9808052.6, and is relevant for novelty under Article 54(3) EPC.

It is an objective of the present invention to provide a orally administerable pharmaceutical products and suppositories that alleviates some of the above mentioned problems relating to drug delivery. It is a further objective of the invention to provide methods of fabricating such orally administrable pharmaceutical products and suppositories.

According to a first aspect, this invention provides an orally administrable pharmaceutical product comprising porous and/or polycrystalline silicon.

An orally administrable pharmaceutical product must have a size, shape, and surface properties that make it suitable for oral consumption by a patient.

Preferably the structure of the porous and/or polycrystalline silicon, used in the orally administrable product, is such that the porous and/or polycrystalline silicon is corrodable in a patient's body; more preferably the porous and/or polycrystalline silicon is corrodable in the patient's intestines; yet more preferably the porous and/or polycrystalline silicon is corrodable in the patient's colon.

Throughout this specification the term "patient" means a human or animal to which a pharmaceutical product is to be administered.

Corrosion of the porous and/or polycrystalline silicon may result from interaction of the porous and/or polycrystalline silicon with the gastrointestinal environment.

Advantageously the orally administrable pharmaceutical product comprises a beneficial substance. More advantageously the arrangement of the beneficial substance and of the porous and/or polycrystalline silicon is such that, once orally administered, the corrosion of the porous and/or polycrystalline silicon in a patient's body results in release of the beneficial substance.

The orally administrable pharmaceutical product may comprise a plurality of beneficial substances.

Throughout this specification the term "beneficial substance" means a substance that has an overall beneficial effect upon the patient. The term "beneficial substance" includes drugs and minerals. The term "beneficial substance" also includes substances that may be, to some extent, toxic to the patient provided the overall effect upon the patient was beneficial. For example anti-cancer drugs are often, to some degree, toxic to the patient.

Preferably the orally administrable pharmaceutical product further comprises an excipient coating or shell that is arranged such that the beneficial substance and porous and/or polycrystalline silicon are all located substantially within the shell or coating.

The excipient coating or shell makes oral consumption of the pharmaceutical product more acceptable to the patient by improving the taste or surface texture of the product. The excipient coating may comprise one or more of: sucrose, gelatine, lactose, dextrose, starch, mannitol, cellulose, cellulose derivatives, dicalcium phosphate, tricalcium phosphate, calcium carbonate, calcium sulphate.

Advantageously the excipient coating or shell is formed and has a composition such that the shell or coating is breachable once the pharmaceutical product has been orally administred.

The coating or shell may be breached as a result of interaction between the pharmaceutical product and the gastric tract and/or mouth and/or intestine. The breach of the coating or shell may result from complete digestion of the coating or shell once the pharmaceutical product has been orally administered. The breach may result from tearing or deformation of the coating or shell. The breach of the coating or shell allows exposure of the porous and/or polycrystalline silicon to the gastrointestinal environment and subsequent delivery of the beneficial substance.

More advantageously the excipient coating is formed and has a composition such that, once the pharmaceutical product has been orally administered, the shell or coating is breachable prior to entry into the intestinal tract.

The coating or shell may be breached as a result of interaction between the pharmaceutical product and the gastric tract and/or mouth. The breach of the coating or shell, prior to entry into the intestine, ensures that the porous and/or polycrystalline silicon is exposed to the intestinal environment during its passage through the intestines.

Preferably the orally administrable pharmaceutical product is a capsule and comprises an excipient shell.

Advantageously the orally administrable pharmaceutical product is a tablet or pellet and comprises an excipient coating.

Preferably the orally administrable pharmaceutical product comprises a corrosion catalyst or inhibitor that catalyses or inhibits the corrosion of porous and/or polycrystalline silicon.

The orally administrable pharmaceutical product may comprise an oxidising catalyst comprising one or more of: copper, gold, silver, iron, nickel, zinc, manganese, chromium, or germanium. The orally administrable pharmaceutical product may comprise an alkaline or acidic buffer salt.

The presence of an oxidising catalyst may assist the corrosion of the porous and/or polycrystalline silicon in the gastrointestinal tract; thereby allowing a further degree of control over the location of drug delivery.

Advantageously the orally administrable pharmaceutical product comprises derivatised porous and/or polycrystalline silicon; more advantageosly the derivatised porous and/or polycrystalline silicon comprises Si-C and/or Si-O-C bonding.

For the absence of doubt, derivatized porous silicon is porous silicon having a substantially monomolecular layer that is covalently bonded to at least part of the porous silicon surface.

Preferably the orally administrable pharmaceutical product comprises porous silicon having a porosity greater than 30%; more preferably the product comprises porous silicon having a porosity greater than or equal to 70%.

The porous and/or polycrystalline silicon may have a structure such that the porous and/or polycrystalline silicon exhibits an enteric surface; an enteric surface being a surface that exhibits substantially no corrosion when immersed in the gastric environment.

Advantageously the porous and/or polycrystalline silicon has a structure such that the porous and/or polycrystalline silicon exhibits substantially no corrosion when immersed in a simulated gastric environment for periods less than 10 minutes; more preferably when immersed for periods less than 1 hour of immersion; yet more preferably when immersed for periods less than 20 hours of immersion.

Preferably the beneficial substance comprises a colonic drug; more advantageously the colonic drug comprises one or more of: an analgesic polypeptide, a contraceptive polypeptide, an oral vaccine, a growth hormone, insulin, an interferon, an erythropoietin, and an interleukin.

Porous and/or polycrystalline silicon is not appreciably corroded in the gastric tract; and it is thus possible to use porous and/or polycrystalline silicon to protect colonic drugs from the gastric environment. Further, porous and/or polycrystalline silicon is corroded in the intestine. This makes porous and/or polycrystalline silicon an ideal carrier for colonic drugs; once the drug has passed through the stomach, the porous and/or polycrystalline silicon corrodes to release the drug. The fact that porous and/or polycrystalline silicon is not corroded in the gastric environment is unexpected because corrosion of porous and/or polycrystalline silicon has been shown in simulated body fluids. It is surprising that porous and/or polycrystalline silicon is corroded in simulated body fluids but is uncorroded, or shows minimal corrosion, in the much harsher gastric environment.

Advantageously the pharmaceutical product comprises an electronic circuit. The electronic circuit may comprise a silicon integrated circuit. The electronic circuit may also comprise a radio receiver and/or transmitter.

The electronic circuit may form at least part of a timing device. The timing device may be used to control the time of release, of a beneficial substance, after administration. The timing device may also be used to control release of a beneficial substance at intervals, as the pharmaceutical product passes through the gastrointestinal tract.

Porous silicon is formed by anodising bulk crystalline silicon and so it is relatively easy to associate the porous silicon with a silicon integrated circuit; the circuit may be formed from the same sample of bulk crystalline silicon from which the porous silicon was derived.

The presence of a radio transmitter and/or receiver may be advantageous since it may allow control over the release of the drug. The transmitter may be used to send signals to or receive signals from the orally administrable pharmaceutical product. The receiver may be used to receive signals instructing the orally administrable pharmaceutical product to release the drug at an appropriate point in the gastrointestinal tract.

Preferably the orally administrable pharmaceutical product comprises a heating means for heating the porous and/or polycrystalline silicon. The heating means may comprise a magnetic material. The heating means may comprise a means for applying a potential difference across at least part of the porous and/or polycrystalline silicon. The means for applying a potential difference may comprise a layer of conducting material, in electrical contact with the porous and/or polycrystalline silicon; the potential difference being generated by electrochemical interaction between the conducting material, the porous and/or polycrystalline silicon, and the gastrointestinal environment. The layer of conducting material, in contact with the porous and/or polycrystalline silicon, may comprise one or more of: gold, silver, copper, zinc, magnesium, aluminium, and bulk crystalline silicon. The potential difference may also result from the formation of electrical contact between porous and polycrystalline silicon, the porous and polycrystalline silicon each interacting electrochemically with the gastrointestinal environment.

Advantageously the orally administrable pharmaceutical product may comprise a reservoir which contains the beneficial substance; more advantageously the reservoir is at least partly defined by a wall formed from the porous and/or polycrystalline silicon.

Once the pharmaceutical product enters the gastrointestinal tract the porous and/or polycrystalline silicon may form a barrier that separates the reservoir from the gastrointestinal environment. The gastrointestinal environment may corrode the barrier so that the beneficial substance is exposed to the gastrointestinal environment. The pharmaceutical product may comprise a plurality of reservoirs; each reservoir having a porous and/or polycrystalline silicon barrier associated with it. The porous and/or polycrystalline silicon may be fabricated in such a manner that each barrier is corrodable at a different rate so that the contents of the reservoir are released or exposed sequentially with time. Each barrier may have different dimensions, the dimensions of being such that the contents of the reservoir are exposed or released sequentially with time. Each barrier may have different dimensions, the dimensions being such that the contents of the reservoir are exposed or released sequentially with time.

According to a second aspect, this invention provides a method of making an orally administrable pharmaceutical product, the method comprising the step of taking a sample of silicon and processing the silicon to form an orally administrable sample of porous and/or polycrystalline silicon.

Preferably the method of making an orally administrable pharmaceutical product further comprises the step of forming an excipient coating or shell in such a manner that the porous and/or polycrystalline silicon is substantially within the shell or coating, and in such a manner that the excipient shell or coating is breachable once the pharmaceutical product has been orally administered. More preferably the product comprises porous silicon and the processing step comprises the step of anodising a sample of bulk crystalline silicon. Yet more preferably the sample of bulk crystalline silicon is a silicon wafer and the processing step comprises the step of dicing the silicon wafer.

The dicing of the silicon wafer may occur before or after the anodisation step.

Advantageously the method of making an orally administrable pharmaceutical product further comprises the step of introducing a beneficial substance into the porous and/or polycrystalline silicon.

Preferably the method of making an orally administrable pharmaceutical product further comprises the steps (i) of forming a reservoir in such a manner that the reservoir is at least partly bounded by the porous and/or polycrystalline silicon, and (ii) disposing a beneficial substance within the reservoir.

Advantageously the method of making an orally administrable pharmaceutical product comprises the step of forming at least one reservoir.

Preferably the method of making an orally administrable pharmaceutical product comprises the step of introducing an oxidising catalyst, that catalyses the oxidation of the porous and/or polycrystalline silicon, into the porous and/or polycrystalline silicon. More preferably the oxidising catalyst comprises one or more of: copper, gold, silver, iron, nickel, zinc, manganese, chromium, or germanium.

Advantageously the beneficial substance comprises a colonic drug.
More advantageously the colonic drug comprises one or more of: an analgesic polypeptide, a contraceptive polypeptide, an oral vaccine, a growth hormone, insulin, an interferon, an erythropoietin, and an interleukin.

Preferably the pharmaceutical product for oral administration comprises porous silicon and the anodisation is carried out in such a manner that the porous silicon has a porosity greater than 30%; more preferably the anodisation is carried out in such a manner that the porosity is greater than or equal to 70%.

Advantageously the method of making an orally administrable pharmaceutical product comprises the step of forming a silicon integrated circuit.

According to a third aspect, this invention provides a use of a pharmaceutical product comprising porous and/or polycrystalline silicon and a beneficial substance in the manufacture of a medicament for the treatment of intestinal or rectal disease.

According to a fourth aspect, this invention provides a use of a pharmaceutical product comprising porous and/or polycrystalline silicon and a beneficial substance in the manufacture of a medicament for the treatment of intestinal or rectal disease via oral administration of the medicament.

According to a fifth aspect, this invention provides an orally administrable pharmaceutical product comprising porous and/or polycrystalline silicon and a beneficial substance, the product being adapted for use in a method of treatment of a human or animal body.

Preferably the beneficial substance comprises a colonic drug.

According to a sixth aspect, this invention provides a suppository comprising porous and/or polycrystalline silicon.

The suppository may be adapted for rectal, vaginal, or urethral administration. The structure of the porous and/or polycrystalline silicon, used in the suppository, may be such that the porous and/or polycrystalline silicon is corrodable in a patient's body accessed via one of these routes.

Preferably the suppository is adapted for rectal administration.

The suppository should have a shape and dimensions that allows rectal administration. Rectal suppositories may be 20 to 40 mm in length, have a substantially circular cross section and have a taper at each end.

Advantageously the suppository comprises an insertion coating or shell that is arranged such that the porous and/or polycrystalline silicon is located substantially within the insertion coating. More advantageously the insertion coating or shell is such that it melts and/or dissolves and/or is deformed once the suppository has been rectally administered so that the porous and/or polycrystalline silicon is exposed to the anorectal or intestinal environment.

The insertion coating or shell may comprise a substance having a melting temperature between 35°C and 45°C.

Preferably the suppository comprises a beneficial substance. More preferably the suppository comprises a colonic or rectal drug. Yet more preferably the suppository comprises one or more of: an analgesic polypeptide, a contraceptive polypeptide, an oral vaccine, a growth hormone, insulin, an interferon, an erythropoietin, and an interleukin.

For the purposes of this specification the term "rectal drug" means a drug that is best delivered in the rectal region.

Advantageously the suppository comprises a reservoir which contains the beneficial substance. More advantageously the reservoir is at least partly bounded by at least part of the porous and/or polycrystalline silicon.

Preferably the suppository comprises a silicon integrated circuit.

According to a seventh aspect, this invention provides a method of making a rectal. suppository comprising the step of taking a sample of silicon and processing the silicon to form a rectally administrable sample of porous and/or polycrystalline silicon.

The structure of the porous and/or polycrystalline silicon, used in the rectally administrable suppository, may be such that the porous and/or polycrystalline silicon is corrodable in a patient's body parts accessable via the rectum.

Advantageously the method for fabricating a suppository further comprises the step of forming a insertion coating or shell in such a manner that the porous and/or polycrystalline silicon is substantially within the shell or coating, and in such a manner that the insertion shell or coating is breachable once the pharmaceutical product has been rectally administered.

Preferably the processing step, for the preparation of a rectally administrable suppository, comprises the step of anodising a sample bulk crystalline silicon to form porous silicon. More preferably the sample of bulk crystalline silicon is a silicon wafer and the processing step further comprises the step of dicing the silicon wafer. Yet more preferably the anodisation is carried out in such a manner that the porous silicon has a porosity greater than 30%.

Advantageaously the method of making a suppository further comprises the step of introducing a beneficial substance into the porous and/or polycrystalline silicon. More advantageously the beneficial substance comprises a colonic drug and/or an anorectal drug.

Preferably the method of making a suppository further comprises the steps (i) of forming a reservoir in such a manner that the reservoir is at least partly bounded by the porous/and or polycrystalline silicon, and (ii) disposing a beneficial substance within the reservoir. More preferably the beneficial substance comprises a colonic drug and/or an anorectal drug.

Advantageously the method of making a suppository comprises the step of forming a silicon integrated circuit from the bulk crystalline silicon.

According to an eighth aspect, the invention provides a method of administering a beneficial substance to the intestinal tract of an animal or human, the method comprising the steps: (i) orally administering a pharmaceutical product comprising porous and/or polycrystalline silicon and a beneficial substance, and (ii) allowing the pharmaceutical product to pass into the intestinal tract.

According to a ninth aspect, the invention provides a method of administering a beneficial substance to the intestines or rectum of an animal or human, the method comprising the step of rectally administering a pharmaceutical product comprising porous and/or polycrystalline silicon and a beneficial substance.

According to a tenth aspect the invention provides an orally administrable composition comprising porous and/or polycrystalline silicon.

Preferably the orally administrable composition comprises a beneficial substance.

Advantageously the orally administrable composition is capable of dissolving in the intestinal tract of an animal or human to release the beneficial substance, but is substantially insoluble in the gastric tract of the animal or human.

Embodiments of the invention will now be described by way of example only, with reference to the following drawings:
Figure 1 shows a schematic diagram of a pharmaceutical product according to the invention, the product being a reservoir tablet;
Figures 2 to 4 shows schematic diagrams of the fabrication steps involved in the production of the pharmaceutical product shown in figure 1;
Figure 5 shows a schematic diagram of a pharmaceutical product according to the invention, the product being a carrier tablet;
Figure 6 shows a schematic diagram of a pharmaceutical product according to the invention, the product comprising timing device;
Figure 7 shows a schematic diagram of a suppository according to the invention;
Figure 8 shows SEM images of a sample of porous silicon, having a porosity of 75%, that has been immersed in simulated gastric fluid for a number of different time intervals;
Figure 9 shows SEM images of a sample of porous silicon, having a porosity of 75%, that has been immersed in simulated intestinal fluid for a number of different time intervals; and
Figure 10 shows SEM images of a sample of porous silicon, having a porosity of 80%, that has been immersed in simulated intestinal fluid for a number of different time intervals.

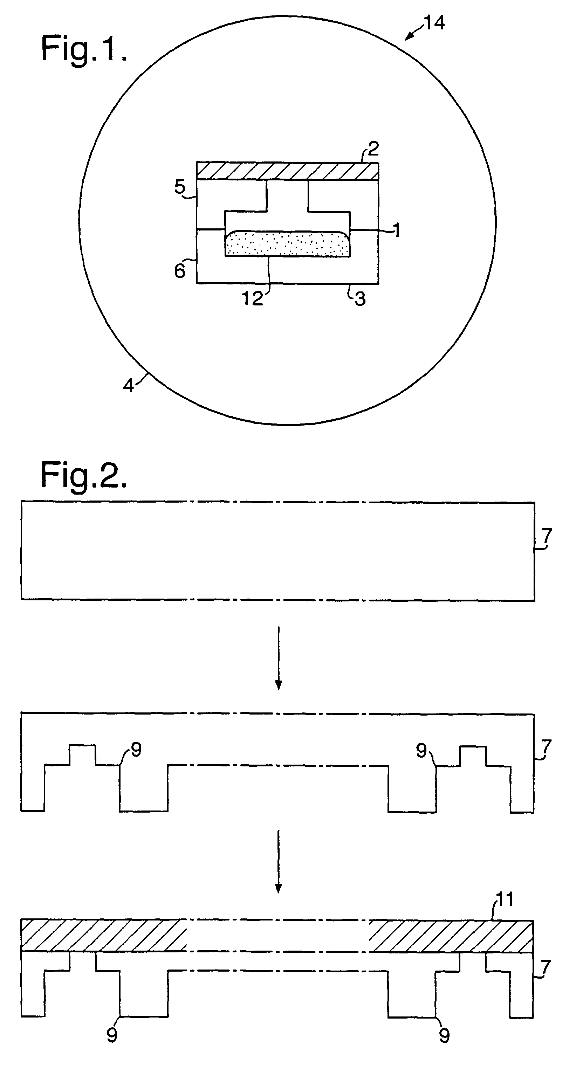

Figure 1 is a schematic diagram of a pharmaceutical product according to the invention. The product is suitable for oral consumption by a patient and is a reservoir tablet 14 having a reservoir 1, a porous silicon barrier 2, a bulk crystalline silicon body 3, and an excipient coating 4. For the purposes of this specification a reservoir tablet is defined as a tablet that comprises at least one reservoir, a beneficial substance being disposed in the or at least one of the reservoirs. The silicon body comprises a first body component 5 and a second body component 6. The reservoir contains a beneficial substance 12.

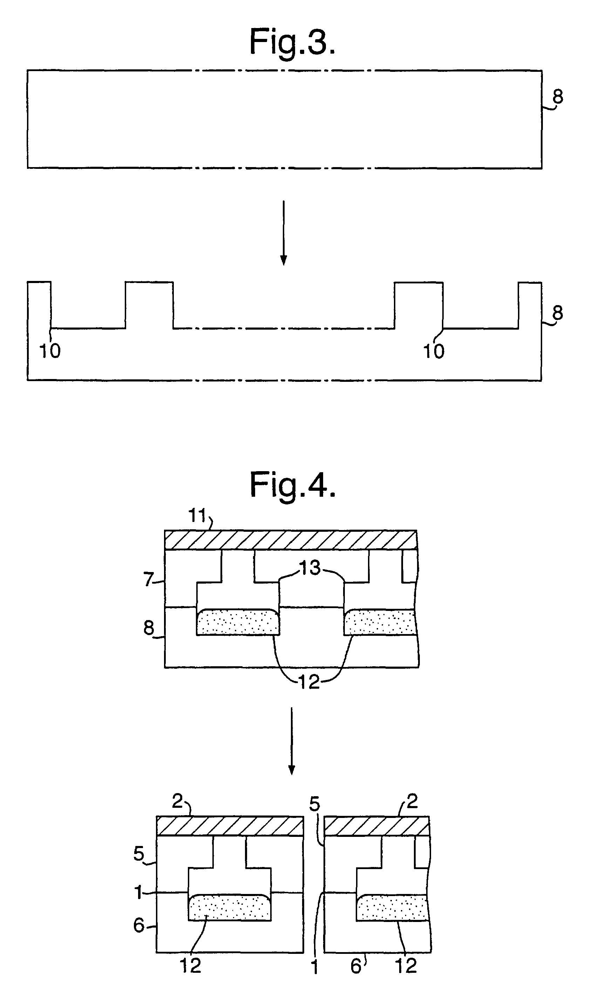

The process by which the reservoir tablet may be fabricated is shown, schematically, in figures 2 to 4. Figure 2 shows a first silicon wafer 7, and figure 3 shows a second silicon wafer 8. The first wafer 7 is micromachined to form a multiplicity of first body components 9, and the second wafer 8 is micromachined to form a multiplicity of second body components 10. One surface of the first wafer is then anodised in aqueous HF, as described in US Patent No. 5,348,618, to form a layer of porous silicon 11. The first and second wafers 7, 8 together with the beneficial substance 12 are assembled and the wafers 7, 8 are bonded together, as shown in figure 4. The beneficial substance may be introduced into the body components 8, 9 by dissolving the substance in a suitable solvent, coating the components 8, 9 with the solvated beneficial substance, and allowing the substance to pass into the components 8, 9 by capillary action. The arrangement of the bonded wafers 7, 8 is such that a multiplicity of reservoirs 13 are formed, each containing the beneficial substance 12. The bonded wafers are then diced to form a multiplicity of silicon bodies 1, as shown in figure 4; each body then being encased in an excipient coating. The dimenstions and surface texture of the reservoir tablet are such that the tablet is suitable for oral administration.

The reservoir tablet is suitable for colonic drugs that must be delivered to the a patient's colon and which must therefore be protected from the gastric environment. The excipient coating is digested by the patient, before it reaches the intestines. The porous silicon barrier is resistant to the gastric environment so that the beneficial substance is able to pass, in a chemically unaltered state, into the intestines. The porous barrier is then corroded resulting in release of the colonic drug. The exact location at which the drug is released depends upon the time taken for the barrier to be corroded, which in turn depends upon the corrosion rate and upon the dimensions of the barrier. The dimensions of the barrier can be altered by altering the duration of the HF anodisation and the conditions under which the etch is performed. A number of factors affect the corrosion rate and these are discussed below.

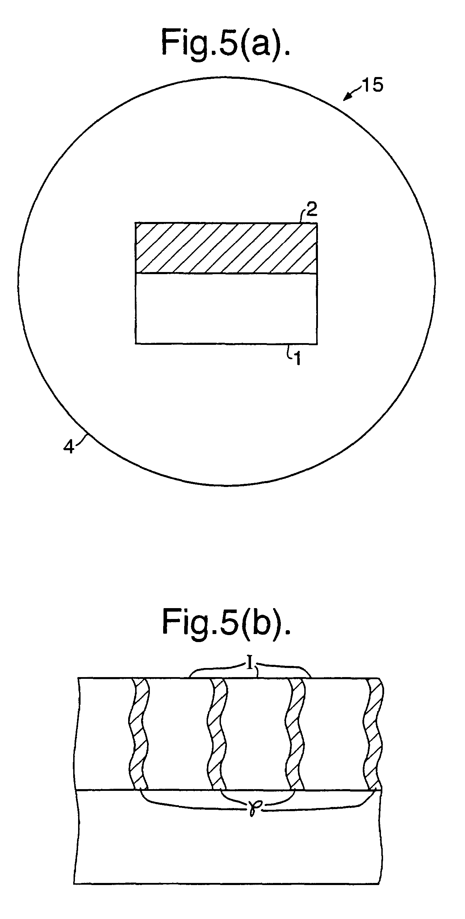

Figure 5 (a) and (b) are schematic diagrams of a pharmaceutical product according to the invention. Figure 5 (a) shows a product suitable for oral administration to a patient; the product is a carrier tablet 15 comprising a bulk crystalline silicon body 1, an excipient substance 4, and a porous silicon carrier region 2. For the purposes of this specification, a carrier tablet is defined as a tablet in which a beneficial substance is stored throughout a porous and/or polycrystalline silicon region, prior to release into a patient. The carrier tablet 15 is fabricated in a similar manner to that by which the reservoir tablet 14 is fabricated. A wafer is porosified by anodising a silicon wafer, the wafer is then diced to form a multiplicity of porous carrier regions 2. Each porous carrier region 2 is then encased in an excipient substance 4. A schematic cross-section of the porous silicon carrier region 2 is shown in figure 5(b). The pores P are separated from each other by inter-pore regions I of silicon. If the pores P are sufficiently close together then the dimensions of the inter-pore regions become so narrow that silicon quantum wires are formed.

The pharmaceutical product, shown in figure 5, further comprises a beneficial substance that is distributed throughout the porous silicon. The beneficial substance may be present in the pores, for example molecules of the beneficial substance may be bonded to the surface of the silicon in which the pores are formed. The distribution of the beneficial substance in the pores may be achieved by placing the porous carrier region 2 in a solution of the beneficial substance, so that the substance passes from the solution into the pores. Alternatively the beneficial substance could be present in the aqueous solution of HF that is used to anodise the bulk silicon body; the beneficial substance being deposited in the pores as they are formed. The beneficial substance may also be introduced into the inter-pore regions by ion implantation of the beneficial substance into the silicon wafer, the wafer is then annealed and anodised to yield a layer of porous silicon.

The carrier tablet may be used to deliver a colonic drug. The excipient coating 4 of the carrier tablet is digested in the patient's stomach. The porous carrier 2 is resistant to the gastric environment so that colonic drug is able to pass into the intestines where corrosion of the porous silicon carrier 2 results in immediate release of the drug. The location and rate at which the drug is released depends on the rate of corrosion of the porous silicon.

The rate of corrosion may be varied by varying the porosity of the porous silicon; high porosities resulting in high corrosion rates. The rate of corrosion may also be increased by the use of a corrosion catalyst or inhibitor.

A further way in which the corrosion rate may be increased is by heating the porous and/or polycrystalline silicon from which the pharmaceutical product is comprised. A heating means comprising a strip of magnetic material such as iron and may be placed in contact with the porous and/or polycrystalline region. A magnetic field may then be applied to the heating means in such a manner that eddy currents are established in the strip. In the case where the pharmaceutical product comprises porous silicon, the heating means may comprise a magnetic material that has been ion implanted into the bulk crystalline silicon from which the porous silicon has been formed. The ion implanted magnetic material may similarly be heated by applying a magnetic field to the porous silicon.

Alternatively the heating means may comprise resistive wire and be connected to a power source, the flow of electric current through the resistive wire causing the porous and/or polycrystalline silicon to be heated. A yet further way in which the porous and/or polycrystalline silicon may be heated is by applying a potential difference across the porous and/or polycrystalline so that an electric current passes through the porous and/or polycrystalline region. The passage of current through the porous and/or polycrystalline silicon heating the region and its surroundings.

A potential difference may be applied across the porous and/or polycrystalline region in a number of ways. Referring again to figure 5, a layer of metal such as gold may be deposited on the porous carrier region 2 or on the bulk crystalline silicon body 1; the more noble of the metal or semiconductor forming the anode when exposed to the GI environment. A similar effect may be achieved by using a silicon body 1 formed from degeneratively doped bulk crystalline silicon, a potential difference being formed between the carrier region 2 and the crystalline body 1 as a result of electrochemical interaction with the GI environment.

To fabricate a sample, in which a metal is in contact with the crystalline silicon body 1, metal is deposited onto one face of the wafer. A good ohmic contact may be achieved, between the wafer and the metal, by thermal sintering of the metal. The uncoated side of the wafer is then anodised to form the layer of porous silicon. To fabricate a sample in which the metal is in contact with the porous carrier region 2, the metal may simply be thermally evaporated onto the porous layer after anodisation.

Figure 5 shows a carrier tablet 15 comprising a porous carrier region 2 and a crystalline body 1. However, it is possible to have a carrier tablet 15 in which the porous carrier region 2 is not in contact with bulk crystalline silicon. This may be achieved by anodisation of a silicon wafer to form a layer of porous silicon and then markedly increasing the current density to a value and for a duration such that the layer of porous silicon becomes detached from the remainder of the wafer. Such a detached layer of porous silicon may similarly be coated with a layer of metal with the intention of generating a potential difference once the tablet is in the GI tract.

The reservoir tablet 14 or the carrier tablet 15, shown in figures 1 and 5, may further comprise a sensor that senses the environment of the tablet. The sensor may sense the pH or temperature of the tablet environment and may be integral with, bonded to, or separate from the silicon body. The sensor can be used to monitor the position of the tablet in the gastrointestinal tract. Once the sensor indicates that the tablet has reached the desired position, the heating element may be heated to release the beneficial substance.

The reservoir or carrier tablets may comprise a radio transmitter and a radio receiver. The radio receiver and transmitter may be integral with or bonded to the silicon body or they may be separate from the silicon body. The radio receiver and transmitter can be used to externally monitor the position of the tablet in the gastrointestinal tract.

The reservoir or carrier tablets may also comprise a control unit in the form of a silicon integrated circuit formed on the silicon body. The control unit may receive input signals from the sensor or from the radio receiver; and may send signals to the power supply of the heating element or to the radio transmitter. The control unit may be used to determine the correct position at which the beneficial substance should be released.

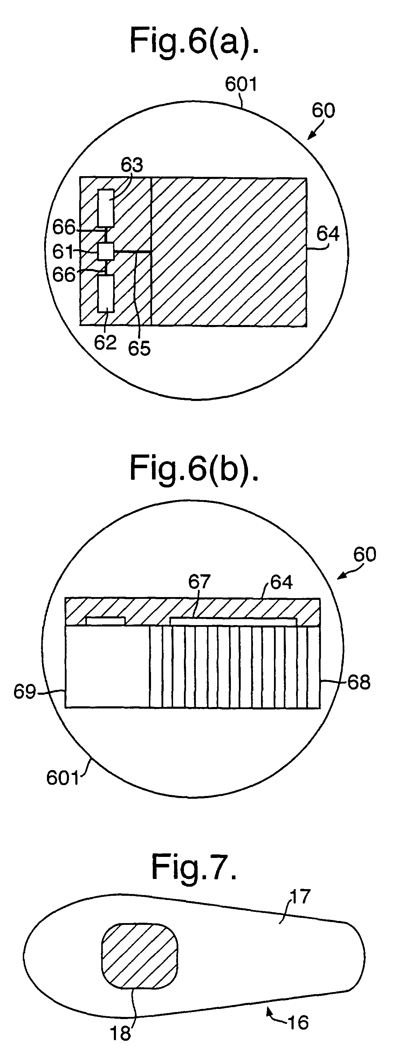

Figure 6a and 6b show a schematic diagrams of an orally administrable pharmaceutical product, generally indicated by 60, according to the invention. Figure 6a shows the top of the product 60 and figure 6b shows one side of the product 60. The product 60 is a carrier tablet and comprises a control unit that includes timing device 61 (for example a ring oscillator structure). The product 60 further comprises a micro battery 62 (for example a lithium based thin film cell with a capacity of >100 µAhr), a thick film cathode 63 (eg 5 - 50µm Au film), a passivation layer 64 (for example a layer of silica or silicon nitride), metallic tracks 65 and 66, a thin film anode 67 (eg 1 - 10 µm Au), a porous silicon carrier region 68, and a bulk silicon region 69. A beneficial substance is distributed throughout the porous carrier region 68. The product 60 further comprises an excpient coating 601. The micro battery 62 and associated circuitry are designed to apply a potential to the porous silicon carrier region 68. The timing device 61 controls the application of the potential difference, the potential either being applied at intervals, or at a predetermined interval from oral administration of the product 60.

The function of the passivation layer 64 is to protect the anode 67, metallic tracks 65, 66, and the micro battery 62. The passivation layer does not obscure the thick film cathode 63 so that, once orally administered, the cathode 63 becomes exposed to the GI environment.

The effect of applying the potential to the porous region 68 may be to promote the release of a beneficial substance. For example the application of a potential may increase corrosion of the porous region 68. Alternatively, if molecules of the beneficial substance are bonded to the surface of the porous silicon, the application of a potential may cause the cleaving of such bonds. (It has been demonstrated (Surface Science 457, L425-L431) that adsorbates containing C-C Π bonds can have their Si-C linkage broken by electrical excitation).

Figure 7 shows a schematic diagram of rectal suppository 16 according to the invention. The suppository has a shape and size that makes it suitable for rectal insertion. The rectal suppository comprises an insertion coating 17 that comprises cocoa butter, a sample of porous silicon 18, and a beneficial substance that is distributed in the porous silicon 18. Part of the cocoa butter may be melted upon insertion of the suppository into the rectum, the melted butter forming a lubrication for insertion. Once inserted the cocoa butter is breached in the rectal region to expose the porous silicon (18) to the rectal or intestinal environment. The porous silicon 18 corrodes in the rectal environment to release the beneficial substance.

Orally administrable pharmaceutical products and suppositories according to the invention may similarly by fabricated using polycrystalline silicon. Polycrystalline silicon may be deposited by standard techniques such as those described in "Polycrystalline Silicon for Integrated Circuit Applications" by T Kamins, published by Kluwer Acad. Publ. 1988. If the orally administrable pharmaceutical product or suppository comprises a beneficial substance then the beneficial substance could be distributed in the grains or grain boundaries of the polycrystalline silicon; alternatively the beneficial substance may be disposed in a reservoir that is at least partially bounded by polycrystalline silicon.

The following experiments demonstrate the utility of porous silicon as an enteric material.

Porous silicon samples were tested in both simulated gastric fluid and simulated intestinal fluid, following the formulations of Cooper et al (Hydrodysis Studies on oleamide in simulated GI fluids, Food Additives + Contaminants 12(6) p769-777 (1995)). The simulated gastric fluid (SGF) was prepared by first diluting 1 M HCl to 0.07M and volume 500cm³. 1.6g of pepsin and 1g of NaCl was then added and the solution incubated at 37°C. The simulated intestinal fluid (SIF) was prepared by dissolving 6.8g of potassium dihydrogen orthophosphate in 250ml of water, then adding 190ml of 0.2M sodium hydroxide, then 400ml of water.

Pancreatin (pig enzymes) was mixed into a stiff paste (10g in 20ml of water) and then added. The pH was adjusted to 7.5 with 0.2M sodium hydroxide and diluted with water to make up 1 litre after 0.5g of cholic acid (bite salts) had also been dissolved. The solution was incubated at 37°C.

Samples of 4.7µm thick porous silicon (70% porosity) were prepared by anodisation of a p⁺ substrate (100 mAcm⁻² for 5 mins in 20% aqueous HF) and subsequent ageing in air. These 70% porosity samples were immersed for 5 hrs, 24 hrs and 67 hrs in SIF, and 5 hrs and 6 weeks in SGF. Significant corrosion was found, by scanning electron microscopy and X-ray analysis, to have occurred in SIF after 5 hrs; the porous silicon being completely corroded after 67 hrs. In SGF the layer became significantly more oxidised within 5 hrs but there was no significant thinning/corrosion even after 6 weeks.

Samples of 40µm thick porous silicon having a porosity of 4%, an average pore width of 100 nm, and a surface area < 1 m²g⁻¹ were prepared from an n⁻ substrate by anodisation (10 mAcm⁻¹ for 5 mins in 20% ethanic HF). These 4% porosity samples exhibited very little corrosion in both SGF and SIF even when the samples were immersed for a period of 67 hours.

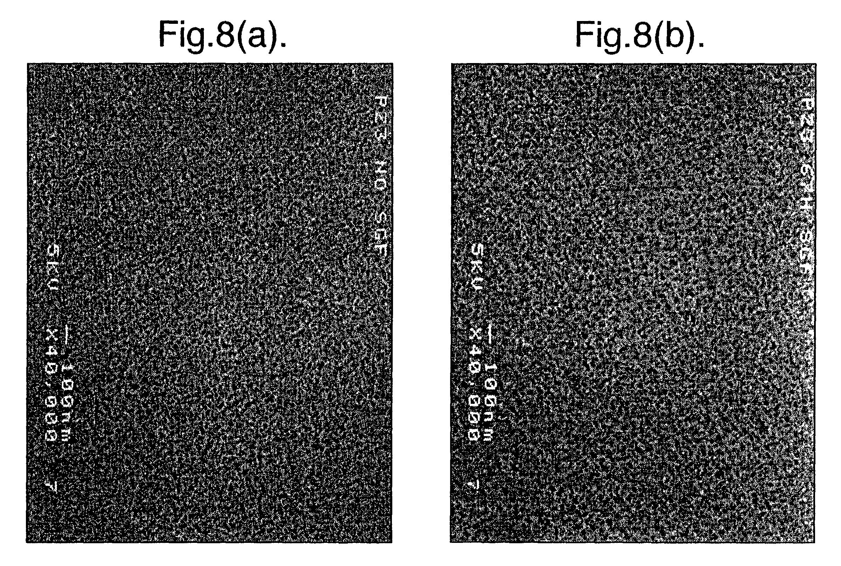

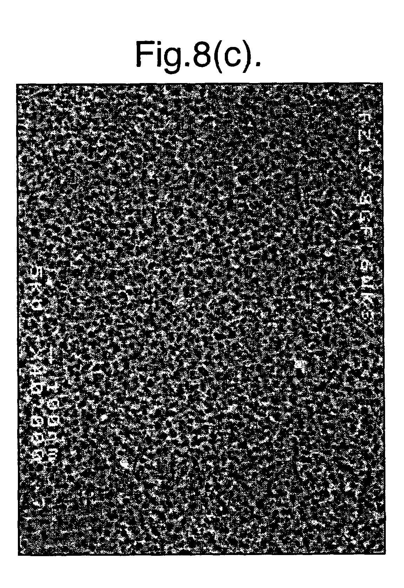

Samples of 75% porosity and 4.3 µm thickness were prepared by anodisation of a p⁺ (0.03 Ωcm) substrate at 100 mAcm⁻² for 1 minute in 20% ethanoic HF (equal volumes of 40 wt% HF and ethanol). Such layers have a surface area of approximately 640 m²g⁻¹ and an average pore width of approximately 10 nm. Wafer segments of these 75% porosity samples were immersed in SGF for periods of up to 6 weeks. Figures 8 (a) to (c) shows SEM images of the surface of the porous silicon sample. Figure 8 (a) shows an image of the sample before immersion, figure 8 (b) shows the sample after incubation for 67 hours, and figure 8 (c) shows the sample after incubation for 6 weeks. At the magnification shown (x 40,000) individual pores are discernible. During the first 1 to 10 hours (which corresponds to a typical transit time for the human stomach) of SGF exposure only oxidation of the porous silicon is observed. After 67 hours exposure to SGF the pore size is comparable to that prior to immersion in the SGF. Figure 8 (c) shows the sample after incubation for 6 weeks. In this case there has been a slight increase in the average pore size, an increase that is indicative of some slight corrosion.

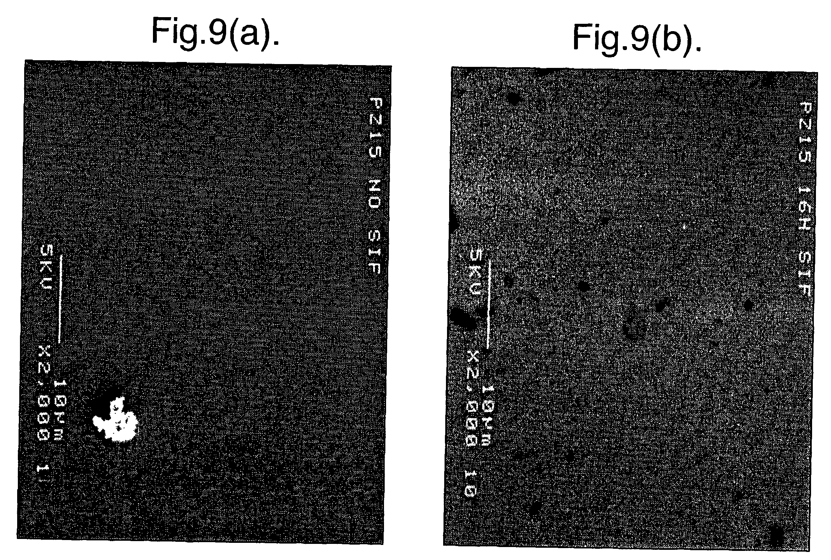

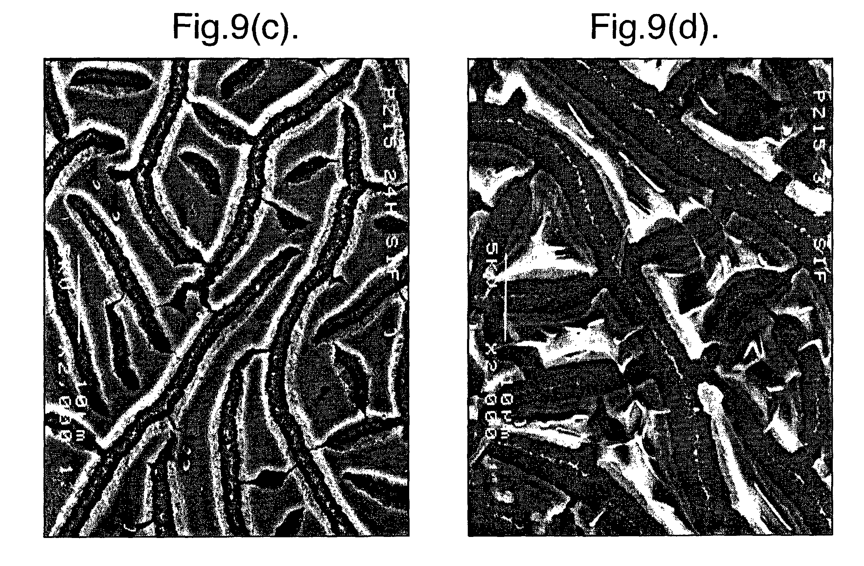

Figures 9 (a) to (d) show SEM images of samples of 75% porous silicon, the preparation of which is described above, after exposure to SIF. The SEM images correspond to a magnification of 2,000, at which the individual pores are not discernible. Figure 9(a) shows a sample prior to exposure to SIF. Figures 9 (b), (c), and (d) show the same sample after exposure for 16, 24, and 30 hours respectively. Figure 9 (b) shows that substantially no corrosion has occurred after 16 hours (the darker patches present on this image are due to organic deposition rather than corrosion). The crazed appearance of the layers in figures 9(c) and 9(d) is due to a non-uniform increase in porosity caused by SIF attack. The structures shown in figures 9 (b) and (c) fragment in air as a result of this increase in porosity.

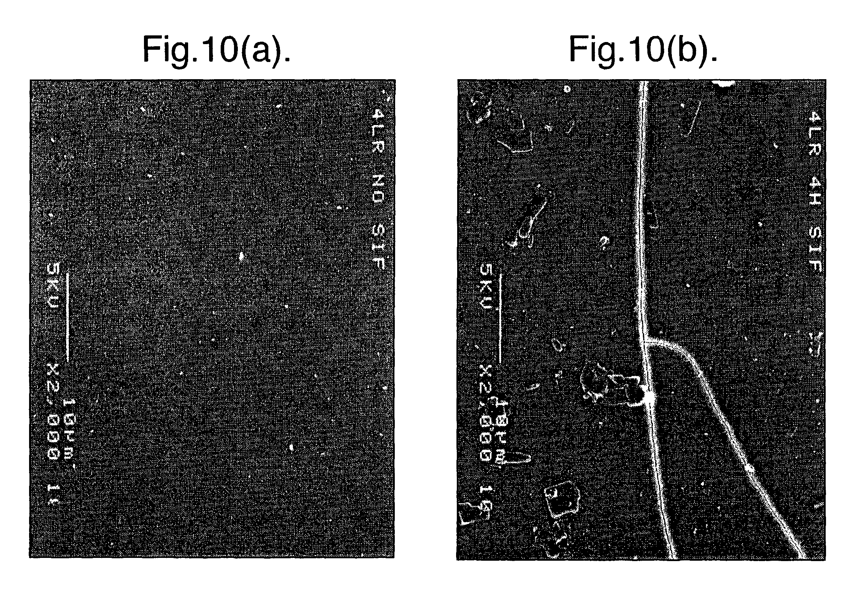

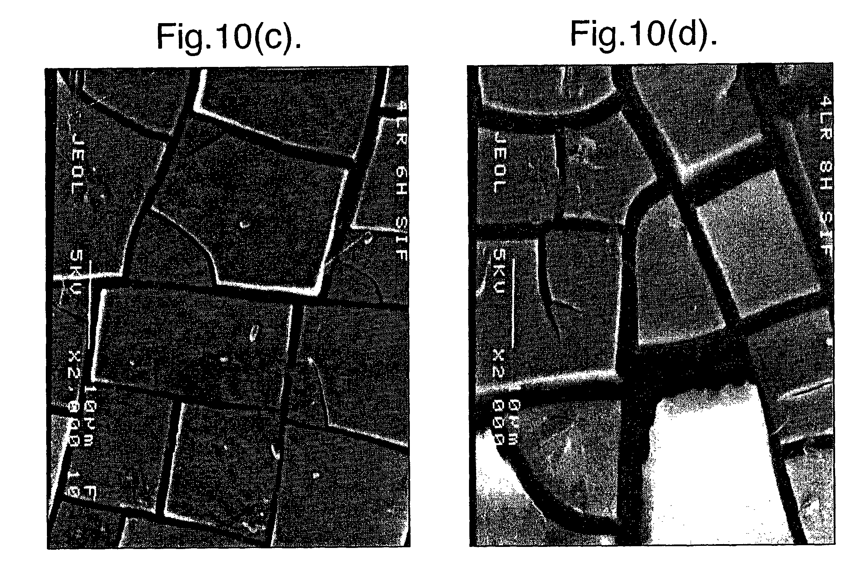

A porous silicon sample having 80% porosity and 4.5 µm thickness were prepared by anodisation of a p⁻ (40 Ωcm) substrate at 20 mAcm⁻² for 5 minutes in 20% ethanoic HF. Such layers have a surface area of 906 m²g⁻¹ and an average pore width of approximately 5nm. These 80% porosity samples were immersed in SIF for 2, 4, 6, 8, and 24 hours. Figure 10 shows SEM images (x 2,000 magnification); figure 10(a) shows the sample prior to immersion in SIF, figures 10 (b), (c), and (d) shows the sample after 4, 6, and 8 hours respectively. Figure 10 (b) shows that there is sufficient corrosion occurring in SIF within only 4 hours to initiate isolated crack propagation during subsequent drying. This contrasts with figure 10 (b) in which no corrosion had occurred after 16 hours in SIF. Substantial corrosion then occurs between 4 and 8 hours (Figures 10 (c) and (d)) of immersion in SIF.

Tests involving immersion in SIF have also been performed on samples that have been pre-treated by immersion in SGF for 1 hour. These tests showed that SIF induced corrosion was not inhibited by prior contact with the simulated gastric environment.

The rate of corrosion of the porous silicon in SIF can thus be controlled by controlling the microstructure of the porous silicon. Bulk crystalline silicon and macroporous silicon (having 4% porosity) exhibiting very low rates of corrosion and whereas high porosity (>70%) mesoporous silicon exhibits high corrosion rates. Transit times are 1 to 10 hours for the stomach, 3 to 4 hours for the small intestine, and 20 to 30 hours for the large intestine (Colonic Drug Targeting Vol 6, No 2, p 129 to 149 (1998)). Figures 9 and 10 show that corrosion can be timed to commence prior to entry of the pharmaceutical product into the large intestine or at the latter stages of colon transport.

## Claims

1. An orally administrable pharmaceutical product comprising: porous and/or polycrystalline silicon, and an excipient coating or shell; the excipient coating or shell being formed and having a composition such that the porous and/or polycrystalline silicon is substantially within the shell or coating, and such that the shell or coating is breachable, once the product has been orally administered, as a result of interaction between the pharmaceutical product and the gastric tract and/or mouth and/or intestine.

2. A pharmaceutical product according to claim 1 **characterised in that** the product comprises a beneficial substance.

3. A pharmaceutical product according to Claim 1 wherein the excipient coating is formed and has a composition such that, once the pharmaceutical product has been orally administered, the shell or coating is breachable prior to entry into the intestinal tract.

4. A pharmaceutical product according to Claim 1 wherein the excipient coating or shell comprises one or more of: sucrose, gelatine, lactose, dextrose, starch, mannitol, cellulose, cellulose derivatives, dicalcium phosphate, tricalcium phosphate, calcium carbonate, calcium sulphate.

5. A pharmaceutical product according to Claim 1 wherein the product is a capsule and comprises an excipient shell.

6. A pharmaceutical product according to Claim 1 wherein the product is a tablet and comprises an excipient coating.

7. A pharmaceutical product according to Claim 2 wherein the beneficial substance comprises a colonic drug.

8. A pharmaceutical product according to Claim 1 wherein the product comprises an oxidising catalyst that catalyses the oxidation of porous and/or polycrystalline silicon.

9. A pharmaceutical product according to Claim 8 wherein the oxidising catalyst comprises one or more of: copper, gold, or silver, iron, nickel, zinc, manganese, chromium, or germanium.

10. A pharmaceutical product according to Claim 1 wherein the porous and/or polycrystalline silicon comprises derivatised porous and/or polycrystalline silicon.

11. A pharmaceutical product according to Claim 10 wherein the derivatised porous and/or polycrystalline silicon comprises one or both of: Si-C bonding and Si-O-C bonding.

12. A pharmaceutical product according to Claim 7 wherein the colonic drug comprises one or more of: an analgesic polypeptide, a contraceptive polypeptide, an oral vaccine, a growth hormone, insulin, an interferon, an erythropoietin, and an interleukin.

13. A pharmaceutical product according to Claim 1 wherein the product comprises porous silicon.

14. A pharmaceutical product according to Claim 13 wherein the product comprises porous silicon having a porosity greater than 30%.

15. A pharmaceutical product according to Claim 14 wherein the product comprises porous silicon having a porosity greater than or equal to 70%.

16. A pharmaceutical product according to Claim 1 wherein the porous and/or polycrystalline silicon has a structure such that the porous and/or pollycrystalline silicon forms an enteric surface.

17. A pharmaceutical product according to Claim 1 wherein the product further comprises an electronic circuit.

18. A pharmaceutical product according to Claim 1 wherein the product further comprises a heating means for heating the porous and/or polycrystalline silicon.

19. A pharmaceutical product according to Claim 18 wherein the heating means comprises a magnetic material.

20. A pharmaceutical product according to Claim 18 wherein the heating means comprises an electrical power supply.

21. A pharmaceutical product according to Claim 20 wherein the heating means comprises a means for applying a potential difference across at least part of the porous and/or polycrystalline silicon.

22. A pharmaceutical product according to Claim 17 wherein the pharmaceutical product further comprises a silicon integrated circuit.

23. A pharmaceutical product according to Claim 1 wherein the product comprises a reservoir which contains the beneficial substance.

24. A pharmaceutical product according to Claim 23 wherein the product comprises a plurality of reservoirs.

25. A pharmaceutical product according to Claim 23 wherein the reservoir is at least partly bounded by at least part of the porous and/or polycrystalline silicon.

26. A method of making an orally administrable pharmaceutical product, the method comprising the step of taking a sample of silicon and processing the silicon to form an orally administrable sample of porous and/or polycrystalline silicon; the method further comprising the step of forming an excipient coating or shell in such a manner that the porous and/or polycrystalline silicon is substantially within the shell or coating, and in such a manner that the excipient shell or coating is breachable, once the pharmaceutical product has been orally administered, as a result of interaction between the pharmaceutical product and the gastric tract and/or mouth and/or intestine.

27. A method according to Claim 26 wherein the product comprises porous silicon and the processing step comprises the step of anodising a sample of bulk crystalline silicon to form the porous silicon.

28. A method according to Claim 27 wherein the sample of bulk crystalline silicon is a silicon wafer and the processing step further comprises the step of dicing the silicon wafer.

29. A method according to Claim 26 wherein the method further comprises the step of introducing a beneficial substance into the porous and/or polycrystalline silicon.

30. A method according to Claim 26 wherein the method further comprises the steps (i) of forming a reservoir in such a manner that the reservoir is at least partly bounded by the porous/and or polycrystalline silicon, and (ii) disposing a beneficial substance within the reservoir.

31. A method according to Claim 30 wherein the method comprises the step of forming at least one reservoir.

32. A method according Claim 26 wherein the method further comprises the step of introducing an oxidising catalyst, that catalyses the oxidation of the porous and/or polycrystalline silicon, into the porous and/or polycrystalline silicon.

33. A method according to Claim 32 wherein the oxidising catalyst comprises one or more of: copper, gold, silver, iron, nickel, zinc, manganese, chromium, or germanium.

34. A method according to Claim 29 or Claim 30 wherein the beneficial substance comprises a colonic drug.

35. A method according to Claim 34 wherein the colonic drug comprises one or more of: an analgesic polypeptide, a contraceptive polypeptide, an oral vaccine, a growth hormone, insulin, an interferon, an erythropoietin, and an interleukin.

36. A method according to Claim 27 wherein the anodisation is carried out in such a manner that the porous silicon has a porosity greater than 30%.

37. A method according to Claim 36 wherein the anodisation is carried out in such a manner that the porous silicon has a porosity greater than or equal to 70%.

38. A method according to Claim 27 wherein the method further comprises the step of forming a silicon integrated circuit from the bulk crystalline silicon.

39. The use of porous and/or polycrystalline silicon in the preparation of a pharmaceutical product for oral administration to an animal or human body, the pharmaceutical product comprising an excipient coating or shell; the excipient coating or shell being formed and having a composition such that the shell or coating is breachable, once the product has been orally administered, as a result of interaction between the pharmaceutical product and the gastric tract and/or mouth and/or intestine.

40. An orally administrable pharmaceutical product comprising porous and/or polycrystalline silicon and a colonic drug the product being for use in a method of treatment of a human or animal body; the product comprising an excipient coating or shell; the excipient coating or shell being formed and having a composition such that the shell or coating is breachable, once the product has been orally administered, as a result of interaction between the pharmaceutical product and the gastric tract and/or mouth and/or intestine.

41. A suppository comprising porous and/or polycrystalline silicon, the suppository being adapted for rectal administration; the suppository comprising an insertion coating or shell that is arranged such that the porous and/or polycrystalline silicon is located substantially within the insertion shell or coating; and wherein the composition and construction of the insertion coating or shell is such that it melts and/or dissolves and/or is deformed, once the suppository has been rectally administered to a patient, so that the porous and/or polycrystalline silicon is exposed to the patient's anorectal or intestinal environment.

42. A suppository according to Claim 41 wherein the suppository comprises a beneficial substance.

43. A suppository according to Claim 42 wherein the beneficial substance is a colonic or anorectal drug.

44. A suppository according to Claim 42 wherein the beneficial substance comprises one or more of: an analgesic polypeptide, a contraceptive polypeptide, an oral vaccine, a growth hormone, insulin, an interferon, an erythropoietin, and an interleukin.

45. A suppository according to Claim 42 wherein the beneficial substance is a anorectal drug.

46. A suppository according to Claim 42 wherein the suppository comprises a reservoir which contains the beneficial substance.

47. A suppository according to Claim 46 wherein the reservoir is at least partly bounded by at least part of the porous and/or polycrystalline silicon.

48. A suppository according to Claim 41 wherein the suppository comprises a silicon integrated circuit.

49. A method of making a rectal suppository comprising the step of taking a sample of a material and processing the sample of material to form a rectally administrable sample; **characterised in that** the material is silicon and processing of the sample of material consists of the step of processing the silicon to form a rectally administrable sample of porous and/or polycrystalline silicon.

50. A method according to Claim 49 wherein the method further comprises the step of forming a insertion coating or shell in such a manner that the porous and/or polycrystalline silicon is substantially within the shell or coating, and in such a manner that the insertion shell or coating is breachable once the pharmaceutical product has been rectally administered.

51. A method according to Claim 49 wherein the suppository comprises porous silicon and the processing step comprises the step of anodising a sample bulk crystalline silicon to form the porous silicon.

52. A method according to Claim 51 wherein the sample of bulk crystalline silicon is a silicon wafer and the processing step further comprises the step of dicing the silicon wafer.

53. A method according to Claim 49 wherein the method further comprises the step of introducing a beneficial substance into the porous and/or polycrystalline silicon.

54. A method according to Claim 49 wherein the method further comprises the steps (i) of forming a reservoir in such a manner that the reservoir is at least partly bounded by the porous/and or polycrystalline silicon, and (ii) disposing a beneficial substance within the reservoir.

55. A method according to Claim 54 wherein the method comprises the step of forming at least one reservoir.

56. A method according to Claim 53 or Claim 54 wherein the beneficial substance comprises a colonic drug or an anorectal drug.

57. A method according to Claim 53 or 54 wherein the beneficial substance comprises one or more of: an analgesic polypeptide, a contraceptive polypeptide, an oral vaccine, a growth hormone, insulin, an interferon, an erythropoietin, and an interleukin.

58. A method according to Claim 51 wherein the anodisation is carried out in such a manner that the porous silicon has a porosity greater than 30%.

59. A method according to Claim 58 wherein the anodisation is carried out in such a manner that the porous silicon has a porosity greater than or equal to 70%.

60. A method according to Claim 51 wherein the method further comprises the step of forming a silicon integrated circuit from the bulk crystalline silicon.

61. The use of porous and/or polycrystalline silicon in the preparation of a suppository.

## Patentansprüche

1. Oral verabreichbares pharmazeutisches Produkt, umfassend:
poröses und/oder polykristallines Silicium und einen Arzneimittelträgerüberzug oder eine Arzneimittelträgerumhüllung, wobei der Arzneimittelträgerüberzug oder die Arzneimittelträgerumhüllung so ausgebildet ist und eine solche Zusammensetzung hat, dass sich das poröse und/oder polykristalline Silicium im wesentlichen innerhalb der Umhüllung oder des Überzugs befindet und dass die Umhüllung oder der Überzug, nachdem das Produkt oral verabreicht worden ist, als Folge einer Wechselwirkung zwischen dem pharmazeutischen Produkt und dem Magentrakt und/oder Mund und/oder Darm aufspaltbar ist.

2. Pharmazeutisches Produkt nach Anspruch 1, **dadurch gekennzeichnet, dass** das Produkt eine vorteilhafte Substanz umfasst.

3. Pharmazeutisches Produkt nach Anspruch 1, wobei der Arzneimittelträgerüberzug so ausgebildet ist und eine solche Zusammensetzung hat, dass, wenn das pharmazeutische Produkt oral verabreicht worden ist, die Umhüllung oder der Überzug vor Eintritt in den Darm-Trakt aufspaltbar ist.

4. Pharmazeutisches Produkt nach Anspruch 1, wobei der Arzneimittelträgerüberzug oder die Arzneimittelträgerumhüllung eine oder mehrere der folgenden Verbindungen umfasst: Saccharose, Gelatine, Lactose, Dextrose, Stärke, Mannit, Cellulose, Cellulosederivate, Dicalciumphosphat, Tricalciumphosphat, Calciumcarbonat, Calciumsulfat.

5. Pharmazeutisches Produkt nach Anspruch 1, wobei das Produkt eine Kapsel ist und eine Arzneimittelträgerumhüllung umfasst.

6. Pharmazeutisches Produkt nach Anspruch 1, wobei das Produkt eine Tablette ist und einen Arzneimittelträgerüberzug umfasst.

7. Pharmazeutisches Produkt nach Anspruch 2, wobei die vorteilhafte Substanz ein Dickdarmarzneimittel umfasst.

8. Pharmazeutisches Produkt nach Anspruch 1, wobei das Produkt einen Oxidationskatalysator umfasst, der die Oxidation von porösem und/oder polykristallinem Silicium katalysiert.

9. Pharmazeutisches Produkt nach Anspruch 8, wobei der Oxidationskatalysator eines oder mehrere der folgenden Elemente umfasst: Kupfer, Gold oder Silber, Eisen, Nickel, Zink, Mangan, Chrom oder Germanium.

10. Pharmazeutisches Produkt nach Anspruch 1, wobei das poröse und/oder polykristalline Silicium derivatisiertes poröses und/oder polykristallines Silicium umfasst.

11. Pharmazeutisches Produkt nach Anspruch 10, wobei das derivatisierte poröse und/oder polykristallinie Silicium eine Si-C-Bindung oder Si-O-C-Bindung oder beide aufweist.

12. Pharmazeutisches Produkt nach Anspruch 7, wobei das Dickdarmarzneimittel eine oder mehrere der folgenden Verbindungen umfasst: ein analgetisches Polypeptid, ein kontrazeptives Polypeptid, ein orales Vakzin, ein Wachstumshormon, Insulin, ein Interferon, ein Erythropoietin und ein Interleukin.

13. Pharmazeutisches Produkt nach Anspruch 1, wobei das Produkt poröses Silicium umfasst.

14. Pharmazeutisches Produkt nach Anspruch 13, wobei das Produkt poröses Silicium mit einer Porosität von höher als 30 % umfasst.

15. Pharmazeutisches Produkt nach Anspruch 14, wobei das Produkt poröses Silicium mit einer Porosität von 70 % oder höher umfasst.

16. Pharmazeutisches Produkt nach Anspruch 1, wobei das poröse und/oder polykristalline Silicium eine solche Struktur hat, dass das poröse und/oder polykristalline Silicium eine magensaftresistente Oberfläche bildet.

17. Pharmazeutisches Produkt nach Anspruch 1, wobei das Produkt außerdem eine elektronische Schaltung umfasst.

18. Pharmazeutisches Produkt nach Anspruch 1, wobei das Produkt außerdem ein Heizmittel zum Erwärmen des porösen und/oder polykristallinen Siliciums umfasst.

19. Pharmazeutisches Produkt nach Anspruch 18, wobei das Heizmittel ein magnetisches Material umfasst.

20. Pharmazeutisches Produkt nach Anspruch 18, wobei das Heizmittel eine Versorgung mit elektrischer Energie umfasst.

21. Pharmazeutisches Produkt nach Anspruch 20, wobei das Heizmittel ein Mittel zum Anlegen einer Potentialdifferenz an mindestens einen Teil des porösen und/oder polykristallinen Siliciums umfasst.

22. Pharmazeutisches Produkt nach Anspruch 17, wobei das pharmazeutische Produkt außerdem einen integrierten Siliciumschaltkreis umfasst.

23. Pharmazeutisches Produkt nach Anspruch 1, wobei das Produkt ein Reservoir umfasst, das die vorteilhafte Substanz enthält.

24. Pharmazeutisches Produkt nach Anspruch 23, wobei das Produkt eine Vielzahl von Reservoiren umfasst.

25. Pharmazeutisches Produkt nach Anspruch 23, wobei das Reservoir mindestens teilweise durch mindestens einen Teil des porösen und/oder polykristallinen Siliciums begrenzt ist.

26. Verfahren zur Herstellung eines oral verabreichbaren pharmazeutischen Produktes, wobei bei dem Verfahren als Schritt eine Siliciumprobe genommen und das Silicium unter Bildung einer oral verabreichbaren Probe aus porösem und/oder polykristallinem Silicum verarbeitet wird und wobei bei dem Verfahren außerdem als Schritt ein Arzneimittelträgerüberzug oder eine Arzneimittelträgerumhüllung derart gebildet wird, dass sich das poröse und/oder polykristalline Silicium im wesentlichen innerhalb der Umhüllung oder des Überzugs befindet und derart, dass die Arzneimittelträgerumhüllung oder der Arzneimittelträgerüberzug, wenn das pharmazeutische Produkt oral verabreicht worden ist, als Resultat der Wechselwirkung zwischen dem pharmazeutischen Produkt und dem Magentrakt und/oder Mund und/oder Darm aufspaltbar ist.

27. Verfahren nach Anspruch 26, wobei das Produkt poröses Silicium umfasst und der Verarbeitungsschritt als Schritt das Anodisieren einer Probe aus kristallinem Siliciummassengut unter Bildung des porösen Siliciums umfasst.

28. Verfahren nach Anspruch 27, wobei die Probe aus kristallinem Siliciummassengut ein Siliciumwafer ist und der Verarbeitungsschritt außerdem als Schritt das Zerteilen des Siliciumwafers umfasst.

29. Verfahren nach Anspruch 26, wobei das Verfahren außerdem als Schritt das Einführen einer vorteilhaften Substanz in das poröse und/oder polykristalline Silicium umfasst.

30. Verfahren nach Anspruch 26, wobei bei dem Verfahren außerdem als Schritt (i) ein Reservoir derart gebildet wird, dass das Reservoir mindestens teilweise durch das poröse und/oder polykristalline Silicium begrenzt ist und als Schritt (ii) eine vorteilhafte Substanz im Reservoir angeordnet wird.

31. Verfahren nach Anspruch 30, wobei das Verfahren als Schritt die Bildung mindestens ein Reservoirs umfasst.

32. Verfahren nach Anspruch 26, wobei bei dem Verfahren außerdem als Schritt ein Oxidationskatalysator, der die Oxidation des porösen und/oder polykristallinen Siliciums katalysiert, in das poröse und/oder polykristalline Silicium eingeführt wird.

33. Verfahren nach Anspruch 32, wobei der Oxidationskatalysator eines oder mehrere der folgenden Elemente umfasst: Kupfer, Gold, Silber, Eisen, Nickel, Zink, Mangan, Chrom oder Germanium.

34. Verfahren nach Anspruch 29 oder Anspruch 30, wobei die vorteilhafte Substanz ein Dickdarmarzneimittel umfasst.

35. Verfahren nach Anspruch 34, wobei das Dickdarmarzneimittel eine oder mehrere der folgenden Substanzen umfasst: ein analgetisches Polypeptid, ein kontrazeptives Polypeptid, ein orales Vakzin, ein Wachstumshormon, Insulin, ein Interferon, ein Erythropoietin und ein Interleukin.

36. Verfahren nach Anspruch 27, wobei die Anodisierung derart durchgeführt wird, dass das poröse Silicium eine Porosität von höher als 30 % hat.

37. Verfahren nach Anspruch 36, wobei die Anodisierung derart durchgeführt wird, dass das poröse Silicium eine Porosität von 70 % oder höher hat.

38. Verfahren nach Anspruch 27, wobei das Verfahren außerdem als Schritt die Bildung eines integrierten Siliciumschaltkreises aus kristallinem Siliciummassengut umfasst.

39. Verwendung von porösem und/oder polykristallinem Silicium bei der Herstellung eines pharmazeutischen Produktes zur oralen Verabreichung an einen tierischen oder menschlichen Körper, wobei das pharmazeutische Produkt einen Arzneimittelträgerüberzug oder eine Arzneimittelträgerumhüllung umfasst und der Arzneimittelträgerüberzug oder die Arzneimittelträgerumhüllung so ausgebildet ist und eine solche Zusammensetzung hat, dass die Umhüllung oder der Überzug, nachdem das Produkt oral verabreicht worden ist, als Resultat einer Wechselwirkung zwischen dem pharmazeutischen Produkt und dem Magentrakt und/oder Mund und/oder Darm aufspaltbar ist.

40. Oral verabreichbares pharmazeutisches Produkt, das poröses und/oder polykristallines Silicium und ein Dickdarmarzneimittel umfasst, wobei das Produkt zur Verwendung in einem Verfahren zur Behandlung eines menschlichen oder tierischen Körpers bestimmt ist und das Produkt einen Arzneimittelträgerüberzug oder eine Arzneimittelträgerumhüllung umfasst, der Arzneimittelträgerüberzug oder die Arzneimittelträgerumhüllung so geformt ist und eine solche Zusammensetzung hat, dass die Umhüllung oder der Überzug, nachdem das Produkt oral verabreicht worden ist, als Resultat einer Wechselwirkung zwischen dem pharmazeutischen Produkt und dem Magentrakt und/oder Mund und/oder Darm aufspaltbar ist.

41. Suppositorium, das poröses und/oder polykristallines Silicium umfasst, wobei das Suppositorium für eine rektale Verabreichung angepasst ist und das Suppositorium einen Einführungsüberzug oder eine Einführungsumhüllung aufweist, die so angeordnet ist, dass das poröse und/oder polykristalline Silicium im wesentlichen innerhalb der Einführungsumhüllung oder des Einführungsüberzugs lokalisiert ist, und wobei die Zusammensetzung und der Aufbau des Einführungsüberzugs oder der Einführungsumhüllung derart ist, dass er/sie schmilzt und/oder aufgelöst und/oder verformt wird, nachdem das Suppositorium rektal einem Patienten verabreicht worden ist, so dass das poröse und/oder polykristalline Silicium der anorektalen oder intestinalen Umgebung des Patienten ausgesetzt wird.

42. Suppositorium nach Anspruch 41, wobei das Suppositorium eine vorteilhafte Substanz umfasst.

43. Suppositorium nach Anspruch 42, wobei die vorteilhafte Substanz ein Dickdarmarzneimittel oder ein anorektales Arzneimittel ist.

44. Suppositorium nach Anspruch 42, wobei die vorteilhafte Substanz eine oder mehrere der folgenden Verbindungen umfasst:
ein analgetisches Polypeptid, ein kontrazeptives Polypeptid, ein orales Vakzin, ein Wachstumshormon, Insulin, ein Interferon, ein Erythropoietin und ein Interleukin.

45. Suppositorium nach Anspruch 42, wobei die vorteilhafte Substanz ein anorektales Arzneimittel ist.

46. Suppositorium nach Anspruch 42, wobei das Suppositorium ein Reservoir umfasst, das die vorteilhafte Substanz enthält.

47. Suppositorium nach Anspruch 46, wobei das Reservoir mindestens teilweise durch mindestens einen Teil des porösen und/oder polykristallinen Siliciums begrenzt ist.

48. Suppositorium nach Anspruch 41, wobei das Suppositorium einen integrierten Siliciumschaltkreis umfasst.

49. Verfahren zur Herstellung eines rektalen Suppositoriums, bei dem als Schritt eine Probe eines Materials genommen und die Materialprobe unter Bildung einer rektal verabreichbaren Probe verarbeitet wird, **dadurch gekennzeichnet, dass** das Material Silicium ist und das Verarbeiten der Materialprobe aus dem Verarbeiten des Siliciums unter Bildung einer rektal verabreichbaren Probe aus porösem und/oder polykristallinem Silicum besteht.

50. Verfahren nach Anspruch 49, wobei bei dem Verfahren außerdem als Schritt ein Einführungsüberzug oder eine Einführungsumhüllung derart gebildet wird, dass sich das poröse und/oder polykristalline Silicium im wesentlichen innerhalb der Umhüllung oder des Überzugs befindet, und in einer Weise, dass die Einführungsumhüllung oder der Einführungsüberzug, nachdem das pharmazeutische Produkt rektal verabreicht worden ist, aufspaltbar ist.

51. Verfahren nach Anspruch 49, wobei das Suppositorium poröses Silicium umfasst und der Verarbeitungsschritt als Schritt die Anodisierung einer Probe aus kristallinem Siliciummassengut unter Bildung des porösen Siliciums umfasst.

52. Verfahren nach Anspruch 51, wobei die Probe aus kristallinem Siliciummassengut ein Siliciumwafer ist und der Verarbeitungsschritt außerdem als Schritt das Zerteilen des Siliciumwafers umfasst.

53. Verfahren nach Anspruch 49, wobei das Verfahren außerdem als Schritt das Einführen einer vorteihaften Substanz in das poröse und/oder polykristalline Silicium umfasst.

54. Verfahren nach Anspruch 49, wobei das Verfahren außerdem als Schritt (i) das Bilden eines Reservoirs derart, dass das Reservoir mindestens teilweise durch das poröse und/oder polykristalline Silicium begrenzt ist, und als Schritt (ii) das Anordnen einer vorteilhaften Substanz innerhalb des Reservoirs umfasst.

55. Verfahren nach Anspruch 54, wobei das Verfahren außerdem als Schritt die Bildung mindestens eines Reservoirs umfasst.

56. Verfahren nach Anspruch 53 oder Anspruch 54, wobei die vorteilhafte Substanz ein Dickdarmarzneimittel oder ein anorektales Arzneimittel umfasst.

57. Verfahren nach Anspruch 53 oder 54, wobei die vorteilhafte Substanz eine oder mehrere der folgenden Verbindungen umfasst:
ein analgetisches Polypeptid, ein kontrazeptives Polypeptid, ein orales Vakzin, ein Wachstumshormon, Insulin, ein Interferon, ein Erythropoietin und ein Interleukin.

58. Verfahren nach Anspruch 51, wobei die Anodisierung derart durchgeführt wird, dass das poröse Silicium eine Porosität von höher als 30 % hat.

59. Verfahren nach Anspruch 58, wobei die Anodisierung derart durchgeführt wird, dass das poröse Silicium eine Porosität von 70 % oder höher hat.

60. Verfahren nach Anspruch 51, wobei das Verfahren außerdem als Schritt die Bildung eines integrierten Siliciumschaltkreises aus kristallinem Siliciummassengut umfasst.

61. Verwendung von porösem und/oder polykristallinem Silicium bei der Herstellung eines Suppositoriums.

## Revendications

1. Produit pharmaceutique destiné à l'administration orale comprenant du silicium poreux et/ou polycristallin et un enrobage ou une enveloppe à base d'excipient ; l'enrobage ou l'enveloppe à base d'excipient ayant une forme et une composition telles que le silicium poreux et/ou polycristallin se retrouve essentiellement enfermé à l'intérieur de l'enveloppe ou de l'enrobage, et telles que l'enveloppe ou l'enrobage est susceptible de se rompre, après l'administration orale du produit, suite aux interactions entre le produit pharmaceutique et le tractus gastrique et/ou la bouche et/ou l'intestin.

2. Produit pharmaceutique selon la revendication 1, **caractérisé en ce que** le produit comprend une substance bienfaisante.

3. Produit pharmaceutique selon la revendication 1, dans lequel l'enrobage à base d'excipient a une forme et une composition telles que, après l'administration orale du produit pharmaceutique, l'enveloppe ou l'enrobage est susceptible de se rompre préalablement à l'entrée dans le tractus intestinal.

4. Produit pharmaceutique selon la revendication 1, dans lequel l'enrobage ou l'enveloppe à base d'excipient comprend une ou plusieurs des substances suivantes : saccharose, gélatine, lactose, dextrose, amidon, mannitol, cellulose, dérivés cellulosiques, phosphate de dicalcium, phosphate de tricalcium, carbonate de calcium, sulfate de calcium.

5. Produit pharmaceutique selon la revendication 1, dans lequel ledit produit consiste en une gélule et comprend une enveloppe à base d'excipient.

6. Produit pharmaceutique selon la revendication 1, dans lequel ledit le produit est un comprimé et comprend un enrobage à base d'excipient.

7. Produit pharmaceutique selon la revendication 2, dans lequel la substance bienfaisante comprend un médicament à libération colonique.

8. Produit pharmaceutique selon la revendication 1, comprenant un catalyseur oxydant capable de catalyser l'oxydation du silicium poreux et/ou polycristallin.

9. Produit pharmaceutique selon la revendication 8, dans lequel le catalyseur oxydant comprend un ou plusieurs des éléments suivants : cuivre, or, ou argent, fer, nickel, zinc, manganèse, chrome ou germanium.

10. Produit pharmaceutique selon la revendication 1, dans lequel le silicium poreux et/ou polycristallin comprend du silicium poreux et/ou polycristallin dérivatisé.

11. Produit pharmaceutique selon la revendication 10, dans lequel le silicium poreux et/ou polycristallin dérivatisé comprend une liaison Si-C ou une liaison Si-O-C ou les deux.

12. Produit pharmaceutique selon la revendication 7, dans lequel le médicament à libération colonique comprend un ou plusieurs des éléments suivants : polypeptide à effet analgésique, polypeptide à effet contraceptif, vaccin oral, hormone de croissance, insuline, interféron, érythropoïétine et interleukine.

13. Produit pharmaceutique selon la revendication 1, comprenant du silicium poreux.

14. Produit pharmaceutique selon la revendication 13, comprenant du silicium poreux présentant une porosité supérieure à 30 %.

15. Produit pharmaceutique selon la revendication 14, comprenant du silicium poreux présentant une porosité supérieure ou égale à 70 %.

16. Produit pharmaceutique selon la revendication 1, dans lequel le silicium poreux et/ou polycristallin présente une structure telle que le silicium poreux et/ou polycristallin forme une surface de type entérique.

17. Produit pharmaceutique selon la revendication 1, comprenant, en outre, un circuit électronique.

18. Produit pharmaceutique selon la revendication 1, comprenant, en outre, un dispositif de chauffage destiné à chauffer le silicium poreux et/ou polycristallin.

19. Produit pharmaceutique selon la revendication 18, dans lequel le dispositif de chauffage comprend un matériau magnétique.

20. Produit pharmaceutique selon la revendication 18, dans lequel le dispositif de chauffage comprend une source de courant électrique.

21. Produit pharmaceutique selon la revendication 20, dans lequel le dispositif de chauffage comprend un dispositif permettant d'appliquer une différence de potentiel au travers d'au moins une partie du silicium poreux et/ou polycristallin.

22. Produit pharmaceutique selon la revendication 17, comprenant, en outre, un circuit intégré sur silicium.

23. Produit pharmaceutique selon la revendication 1, comprenant un réservoir qui contient la substance bienfaisante.

24. Produit pharmaceutique selon la revendication 23, comprenant plusieurs réservoirs.

25. Produit pharmaceutique selon la revendication 23, dans lequel le réservoir est au moins partiellement délimité par au moins une partie du silicium poreux et/ou polycristallin.

26. Procédé de préparation d'un produit pharmaceutique destiné à l'administration orale, comprenant une étape de prélèvement d'un échantillon de silicium et de traitement du silicium de façon à préparer un échantillon de silicium poreux et/ou polycristallin destiné à l'administration orale ; le procédé comprenant, en outre, une étape de préparation d'un enrobage ou d'une enveloppe à base d'excipient de façon à ce que le silicium poreux et/ou polycristallin se retrouve sensiblement enfermé à l'intérieur de l'enveloppe ou de l'enrobage, et de façon à ce que l'enveloppe ou l'enrobage à base d'excipient soit susceptible de se rompre après l'administration orale du produit pharmaceutique, suite aux interactions entre le produit pharmaceutique et le tractus gastrique et/ou la bouche et/ou l'intestin.

27. Procédé selon la revendication 26, dans lequel le produit comprend du silicium poreux et dans lequel les étapes de traitement incluent une étape d'anodisation d'un échantillon de silicium cristallin en vrac pour obtenir du silicium poreux.

28. Procédé selon la revendication 27, dans lequel l'échantillon de silicium cristallin en vrac correspond à une plaque de silicium et dans lequel les étapes de traitement comprennent, en plus, une étape de découpage en cubes de la plaque de silicium.

29. Procédé selon la revendication 26, comprenant, en outre, une étape d'introduction d'une substance bienfaisante dans le silicium poreux et/ou polycristallin.

30. Procédé selon la revendication 26, comprenant, en outre, les étapes (i) de fabrication d'un réservoir de façon à ce que le réservoir soit au moins partiellement délimité par le silicium poreux et/ou polycristallin, et (ii) d'introduction d'une substance bienfaisante à l'intérieur du réservoir.

31. Procédé selon la revendication 30, comprenant une étape de fabrication d'au moins un réservoir.

32. Procédé selon la revendication 26, comprenant, en outre, une étape d'introduction d'un catalyseur oxydant, catalysant l'oxydation du silicium poreux et/ou polycristallin, à l'intérieur du silicium poreux et/ou polycristallin.

33. Procédé selon la revendication 32, dans lequel le catalyseur oxydant comprend un ou plusieurs des éléments suivants : cuivre, or, argent, fer, nickel, zinc, manganèse, chrome ou germanium.

34. Procédé selon les revendications 29 ou 30, dans lequel la substance bienfaisante comprend un médicament à libération colonique.

35. Procédé selon la revendication 34, dans lequel le médicament à libération colonique comprend une ou plusieurs des substances suivantes : polypeptide à effet analgésique, polypeptide à effet contraceptif, vaccin oral, hormone de croissance, insuline, interféron, érythropoïétine et interleukine.

36. Procédé selon la revendication 27, dans lequel l'anodisation est pratiquée de telle sorte que le silicium poreux présente une porosité supérieure à 30 %.

37. Procédé selon la revendication 36, dans lequel l'anodisation est pratiquée de telle sorte que le silicium poreux présente une porosité supérieure ou égale à 70 %.

38. Procédé selon la revendication 27, comprenant, en outre, une étape de fabrication d'un circuit intégré sur silicium à partir du silicium cristallin en vrac.

39. Utilisation de silicium poreux et/ou polycristallin dans la préparation d'un produit pharmaceutique destiné à l'administration orale chez un organisme animal ou humain, le produit pharmaceutique comprenant un enrobage ou une enveloppe à base excipient ; l'enrobage ou l'enveloppe à base d'excipient ayant une forme et une composition telles que l'enveloppe ou l'enrobage est susceptible de se rompre, après l'administration du produit par voie orale, suite aux interactions entre le produit pharmaceutique et le tractus gastrique et/ou la bouche et/ou l'intestin.

40. Produit pharmaceutique destiné à l'administration orale comprenant du silicium poreux et/ou polycristallin et un médicament à libération colonique, le produit étant destiné à être utilisé dans le cadre d'un procédé de traitement d'un organisme humain ou animal ; le produit comprenant un enrobage ou une enveloppe à base d'excipient ; l'enrobage ou l'enveloppe à base d'excipient ayant une forme et une composition telles que l'enveloppe ou l'enrobage est susceptible de se rompre, après l'administration du produit par voie orale, suite aux interactions entre le produit pharmaceutique et le tractus gastrique et/ou la bouche et/ou l'intestin.

41. Suppositoire comprenant du silicium poreux et/ou polycristallin, suppositoire adapté à l'administration par voie rectale ; le suppositoire comprenant un enrobage ou une enveloppe facilitant l'insertion arrangé de façon à ce que le silicium poreux et/ou polycristallin se retrouve sensiblement enfermé à l'intérieur de l'enveloppe ou de l'enrobage facilitant l'insertion ; et dans lequel la composition et le mode de fabrication de l'enrobage ou de l'enveloppe facilitant l'insertion sont tels que l'enrobage ou l'enveloppe puisse fondre et/ou se dissoudre et/ou se déformer, après l'administration du suppositoire à un patient par voie rectale, de façon à ce que le silicium poreux et/ou polycristallin soit exposé à l'environnement ano-rectal ou intestinal du patient.

42. Suppositoire selon la revendication 41, comprenant une substance bienfaisante.

43. Suppositoire selon la revendication 42, dans lequel la substance bienfaisante est un médicament à libération colonique ou ano-rectale.

44. Suppositoire selon la revendication 42, dans lequel la substance bienfaisante comprend une ou plusieurs des substances suivantes : polypeptide à effet analgésique, polypeptide à effet contraceptif, vaccin oral, hormone de croissance, insuline, interféron, érythropoïétine et interleukine.

45. Suppositoire selon la revendication 42, dans lequel la substance bienfaisante est un médicament à libération ano-rectale.

46. Suppositoire selon la revendication 42, comprenant un réservoir qui contient la substance bienfaisante.

47. Suppositoire selon la revendication 46, dans lequel le réservoir est au moins partiellement délimité par au moins une partie du silicium poreux et/ou polycristallin.

48. Suppositoire selon la revendication 41, comprenant un circuit intégré sur silicium.

49. Procédé de fabrication d'un suppositoire rectal comprenant une étape de prélèvement d'un échantillon de matériau et de traitement de l'échantillon de matériau afin de préparer un échantillon destiné à l'administration rectale ; **caractérisé en ce que** le matériau est du silicium et **en ce que** le traitement de l'échantillon de matériau consiste en une étape de traitement du silicium pour préparer un échantillon de silicium poreux et/ou polycristallin destiné à l'administration rectale.

50. Procédé selon la revendication 49, comprenant, en outre, une étape de préparation d'un enrobage ou d'une enveloppe facilitant l'insertion de façon à ce que le silicium poreux et/ou polycristallin se retrouve sensiblement enfermé à l'intérieur de l'enveloppe ou de l'enrobage et de façon à ce que l'enveloppe ou l'enrobage facilitant l'insertion soit susceptible de se rompre après l'administration du produit pharmaceutique par voie rectale.

51. Procédé selon la revendication 49, dans lequel le suppositoire comprend du silicium poreux et dans lequel les étapes de traitement comprennent une étape d'anodisation d'un échantillon de silicium cristallin en vrac pour donner du silicium poreux.

52. Procédé selon la revendication 51, dans lequel l'échantillon de silicium cristallin en vrac est une plaque de silicium et dans lequel les étapes de traitement comprennent, en outre, une étape de découpage en cubes de la plaque de silicium.

53. Procédé selon la revendication 49, comprenant, en outre, une étape d'introduction d'une substance bienfaisante dans le silicium poreux et/ou polycristallin.

54. Procédé selon la revendication 49, comprenant, en outre, des étapes (i) de fabrication d'un réservoir de façon à ce que le réservoir soit au moins partiellement délimité par le silicium poreux et/ou polycristallin, et (ii) d'introduction d'une substance bienfaisante à l'intérieur du réservoir.

55. Procédé selon la revendication 54, comprenant une étape de fabrication d'au moins un réservoir.

56. Procédé selon les revendications 53 ou 54, dans lequel la substance bienfaisante comprend un médicament à libération colonique ou ano-rectale.

57. Procédé selon les revendications 53 ou 54, dans lequel la substance bienfaisante comprend une ou plusieurs des substances suivantes : polypeptide à effet analgésique, polypeptide à effet contraceptif, vaccin oral, hormone de croissance, insuline, interféron, érythropoïétine et interleukine.

58. Procédé selon la revendication 51, dans lequel l'anodisation est pratiquée de façon à ce que le silicium poreux présente une porosité supérieure à 30 %.

59. Procédé selon la revendication 58, dans lequel l'anodisation est pratiquée de façon à ce que le silicium poreux présente une porosité supérieure ou égale à 70 %.

60. Procédé selon la revendication 51, comprenant, en outre, une étape de fabrication d'un circuit intégré sur silicium à partir de silicium cristallin en vrac.

61. Utilisation de silicium poreux et/ou polycristallin dans la préparation d'un suppositoire.
